# EUROPEAN PATENT APPLICATION

(11) **EP 4 534 008 A1**
(43) Date of publication of application: **09.04.2025**
(21) Application number: 23818219.0
(22) Date of filing: 21.08.2023
(51) Int. Cl.: A61B 5/1473, A61B 5/145, A61B 5/00

(54) **ANALYTE SENSOR**

(71) Applicant: Shanghai United Imaging Microelectronics Technology Co., Ltd., Jiading District Shanghai 201800 (CN)
(72) Inventor: LIU, Wei, Shanghai 201800 (CN)
(74) Representative: Viering, Jentschura & Partner mbB Patent- und Rechtsanwälte
(86) International application number: PCT/CN2023/114039
(87) International publication number: WO 2025/039161

(57) **Abstract**

An analyte sensor (100) is provided. The analyte sensor (100) includes a probe (2) and a circuit board (3), a connecting portion (5) is provided on either or both of a first end of the probe (2) and a conductive contact area (301) of the circuit board (3), the first end of the probe (2) is connected to the conductive contact area (301) of the circuit board (3) by the connecting portion (5), and the connecting portion (5) and the first end of the probe (2) are integrally formed or the connecting portion (5) and the conductive contact area (301) of the circuit board (3) are integrally formed.

## Description

### TECHNICAL FIELD

The present invention generally relates to a field of electronic medical technology, and in particular, to an analyte sensor.

### BACKGROUND

An analyte sensor is a detection device that converts a concentration of a bioanalytical substance into a measurable parameter such as an electrical signal, and in particular, an analyte sensor for real-time detection of glucose concentration is usually called a continuous glucose monitoring system. The continuous glucose monitoring system analyzes a concentration of glucose by a probe implanted in subcutaneous tissue of human body and transmits a converted signal to a processing circuit on a transmitter circuit board to accomplish continuous monitoring of the concentration of glucose.

The probe is a thin sheet of material with a certain shape. The probe and the processing circuit belong to different modules, signal transmission between the probe and the processing circuit requires a physical electrical connection, and quality of the electrical connection directly affects reliability of the signal transmission, which in turn affects final monitoring results. Therefore, a simple, robust, and efficient electrical connection scheme is essential to improve signal transmission quality of the continuous glucose monitoring system and even the analyte sensor.

### SUMMARY

According to various embodiments of the present invention, an analyte sensor is provided.

The analyte sensor includes a probe and a circuit board, a connecting portion is provided on either or both of a first end of the probe and a conductive contact area of the circuit board, and the first end of the probe is connected to the conductive contact area of the circuit board by the connecting portion. The connecting portion and the first end of the probe are integrally formed, or the connecting portion and the conductive contact area of the circuit board are integrally formed.

In some embodiments, the connecting portion includes a resilient structure, and the first end of the probe is connected to the conductive contact area of the circuit board by the resilient structure.

In some embodiments, the resilient structure includes a spring plate, and the probe is connected to the conductive contact area of the circuit board by the spring plate.

In some embodiments, the resilient structure includes a protrusion, and the probe is connected to the conductive contact area of the circuit board by the protrusion.

In some embodiments, the connecting portion includes a contact structure, and the first end of the probe is connected to the conductive contact area of the circuit board by the contact structure.

In some embodiments, the contact structure includes a convex contact, and the first end of the probe is connected to the conductive contact area of the circuit board by the convex contact.

In some embodiments, the connecting portion provided on the first end of the probe includes a first matching structure, the connecting portion provided on the conductive contact area of the circuit board includes a second matching structure, and the first matching structure is matched and connected to the second matching structure.

In some embodiments, the first matching structure includes a convex structure, and the second matching structure includes a recessed structure; or the first matching structure includes a recessed structure, and the second matching structure includes a convex structure.

In some embodiments, the connecting portion provided on the first end of the probe includes a spring plate and a protrusion, and the first end of the probe is connected to the conductive contact area of the circuit board by the spring plate and the protrusion, respectively; and the connecting portion provided on the conductive contact area of the circuit board includes a convex contact, and the first end of the probe is connected to the conductive contact area of the circuit board by the convex contact.

In some embodiments, the circuit board is provided with a first through hole, a second end of the probe which is not connected to the conductive contact area of the circuit board is bent relative to the first end of the probe, and the second end of the probe passes through the first through hole of the circuit board when the first end of the probe is connected to the conductive contact area of the circuit board.

In some embodiments, analyte sensor further includes a first cover plate and a second cover plate, the probe and the circuit board are provided between the first cover plate and the second cover plate, and the first cover plate and the second cover plate are detachably connected with each other and configured to fix the probe and the circuit board.

In some embodiments, the first cover plate is provided with a clamping slot, which is configured to accommodate and fix the first end of the probe.

In some embodiments, the first cover plate is further provided with a first matching portion, the second cover plate is provided with a second matching portion, and the first matching portion is matched and connected to the second matching portion; and the second matching portion passes through the first through hole, the first matching portion is provided with a second through hole, and the second matching portion is provided with a third through hole; and when the first matching portion is matched and connected to the second matching portion, the second through hole and the third through hole are coaxially disposed, and the second end of the probe passes through the third through hole.

In some embodiments, the second matching portion is further provided with a groove, and a part of the probe between the first end of the probe and the second end of the probe is disposed in the groove.

Details of one or more embodiments of the present invention are set forth in the following accompanying drawings and descriptions. Other features, objectives, and advantages of the present invention become obvious with reference to the description, the accompanying drawings, and the claims.

### BRIEF DESCRIPTION OF THE DRAWINGS

In order to more clearly illustrate the technical solutions in embodiments of the present invention or the conventional art, the accompanying drawings used in the description of the embodiments or the conventional art will be briefly introduced below. It is apparent that, the accompanying drawings in the following description are only some embodiments of the present invention, and other drawings can be obtained by those of ordinary skill in the art from the provided drawings without creative efforts.
FIG. 1 is a schematic diagram of an application scenario of an analyte sensor according to some embodiments.
FIG. 2A and FIG. 2B are schematic diagrams of an analyte sensor according to some embodiments.
FIG. 3 is a schematic diagram of a probe according to some embodiments.
FIG. 4 is a schematic diagram of a probe according to other embodiments.
FIG. 5 is a schematic diagram of a circuit board states according to some embodiments.
FIG. 6 is a schematic diagram of a connecting portion according to some embodiments.
FIG. 7 is a schematic diagram of an analyte sensor according to other embodiments.
FIG. 8 is a schematic diagram of a first cover plate according to some embodiments.
FIG. 9 is a schematic diagram of a first cover plate and a second cover plate according to some embodiments.
FIG. 10 is a schematic diagram of an overall structure of an analyte sensor according to some embodiments.
FIG. 11 is a schematic diagram of a A-A section of FIG. 10.
FIG. 12 is a schematic diagram of a B-B section of FIG. 10.
FIG. 13 is a schematic diagram of an overall structure of an analyte sensor according to other embodiments.
FIG. 14 is a schematic diagram of a C-C section of FIG. 13.
FIG. 15 is a schematic diagram of a D-D section of FIG. 2.

In the figures, 100 represents an analyte sensor, 200 represents an object to be detected, 300 represents a processing terminal, 1 represents a first cover plate, 101 represents a clamping slot, 102 represents a first matching portion, 103 represents a second through hole, 2 represents a probe, 201 represents a spring plate, 202 represents a protrusion, 3 represents a circuit board, 301 represents a conductive contact area, 302 represents a convex contact, 303 represents a first through hole, 4 represents a second cover plate, 401 represents a second matching portion, 402 represents a third through hole, 403 represents a groove, 5 represents a connecting portion, 51 represents a first matching portion, 52 represents a second matching portion.

### DETAILED DESCRIPTION OF THE EMBODIMENT

The technical solutions in embodiments of the present invention will be described clearly and completely below with reference to the accompanying drawings in the embodiments of the present invention. Apparently, the described embodiments are merely some of rather than all of the embodiments of the present invention. All other embodiments acquired by those skilled in the art without creative efforts based on the embodiments of the present invention shall fall within the protection scope of the present invention.

It should be noted that when an assembly is considered to be "arranged on" another assembly, it can be directly arranged on another assembly, or there can be a centered assembly. When an assembly is considered to be "provided on" another assembly, it can be directly provided on another assembly, or there can be a centered assembly at the same time. When an assembly is considered to be "fixed to" another assembly, it can be directly fixed to another assembly, or there can be a centered assembly at the same time.

Unless otherwise defined, all technical and scientific terms used herein have the same meaning as a skilled person in the art would understand. The terminology used in the description of the present invention is for the purpose of describing particular embodiments and is not intended to limit the invention. The term "and/or" as used herein includes any and all combinations of one or more of the associated listed items.

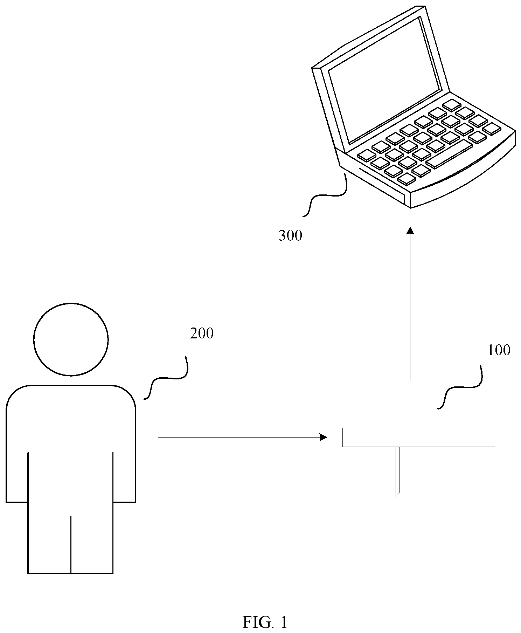

An analyte sensor is a detection device that converts a concentration of a bioanalytical substance into a measurable parameter such as an electrical signal. An analyte sensor for real-time detection of glucose concentration is usually called a continuous glucose monitoring system. The following proceeds with a detailed description in conjunction with the accompanying drawings. FIG. 1 is a schematic diagram of an application scenario of the continuous glucose monitoring system provided in the present embodiment. Referring to FIG. 1, the continuous glucose monitoring system may analyze glucose concentration by a probe implanted in subcutaneous tissue of an object 200 to be detected, convert a signal by a processing circuit, transmit a converted signal to a transmitter circuit board, and send the converted signal to a processing terminal 300 by a network. The processing terminal 300 may receive glucose data of the object 200 to be detected, and give relevant guiding information or suggestions after analysis, which facilitates blood glucose control. The processing terminal 300 may be a cell phone, a tablet, or a computer, etc., and a corresponding monitoring software may be installed in the processing terminal 300. It should be noted that the analyte sensor may also be used to monitor concentration of other bioanalytical substances, such as blood oxygen, white blood cells, platelets and so on.

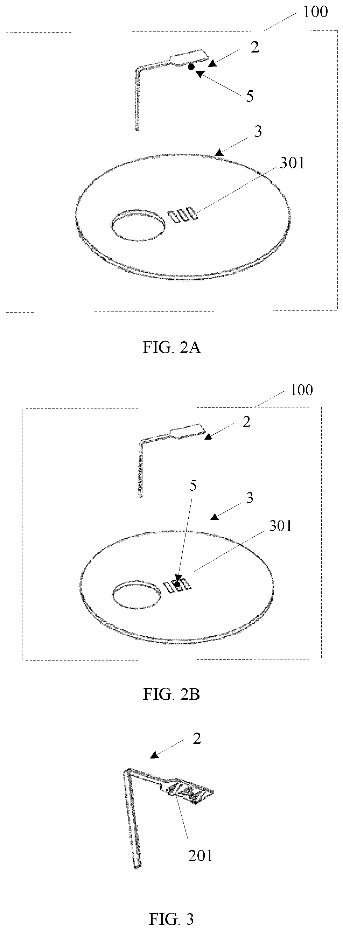

Referring to FIG. 2A and FIG. 2B, the present invention provides an analyte sensor 100, which is applied to the field of electronic medical technology to monitor a level of a bioanalytical substance of an object to be detected. The analyte sensor 100 includes a probe 2 and a circuit board 3. A connecting portion 5 is provided on either or both of a first end of the probe 2 and a conductive contact area 301 of the circuit board 3, and the first end of the probe 2 is connected to the conductive contact area 301 of the circuit board 3 by the connecting portion 5. The connecting portion 5 and the first end of the probe 2 are integrally formed, or the connecting portion 5 and the conductive contact area 301 of the circuit board 3 are integrally formed.

In the related art, electrical connections of an analyte sensor include a Pogo-pin connection, a conductive tape (a structure with layers of conductive rubbers and insulating rubbers) connection, and a connector connection. All of the above three kinds of connections add an additional medium between a probe and a circuit board to complete an electrical connection and signal transmission therebetween. The use of the additional medium to complete the electrical connection between the probe and the circuit board results in transmission losses, risk of connection failure, and increased cost of the additional medium.

In the present invention, the connecting portion 5 is provided on either or both of the first end of the probe 2 and the conductive contact area 301 of the circuit board 3, the connecting portion 5 and the first end of the probe 2 are integrally formed, or the connecting portion 5 and the conductive contact area 301 of the circuit board 3 are integrally formed, and the first end of the probe 2 is connected to the conductive contact area 301 of the circuit board 3 by the connecting portion 5, i.e., the probe 2 is directly connected to the circuit board 3 without the additional connection medium, and a signal acquired by the probe 2 may be directly transmitted to the circuit board 3, which may enhance reliability of the connection between the probe 2 and the circuit board 3, reduce the risk of connection failure, and reduce hardware cost.

It should be noted that the connecting portion 5 may be provided on the first end of the probe 2, be provided on the conductive contact area 30 of the circuit board 3, or be provided on both the first end of the probe 2 and the conductive contact area 30 of the circuit board 3.

Furthermore, a substrate of the probe 2 may be generally an insulating material, such as PI (polyimide), PET (polyethylene glycol terephthalate), and so on. The substrate of the probe 2 may be flexible and elastically deformable, and the substrate may be sequentially coated with a plurality of layers of films, including a conductive film, an insulating film, a bio-enzymatic film, a functional polymer film, and so on.

The circuit board 3 may have a processing circuit and a transmitter circuit, the processing circuit is configured to convert and process the signal acquired by the probe 2 and transmit the converted signal to the transmitter circuit, the processing circuit may include a microprocessor, a power management module, an acquisition and amplification module, an A/D conversion (analog-to-digital conversion) module, a battery, and so on. The transmitter circuit may send the converted signal to the processing terminal 300 by non-contact communication, the transmitter circuit may include a communication module, a battery, and so on. An input end portion of the processing circuit of the circuit board 3 may be provided on a surface of the circuit board 3 in a form of the conductive contact area 301, which may facilitate a direct connection with the first end of the probe 2 for signal transmission.

In some embodiments, the connection portion 5 may include a resilient structure, and the first end of the probe 2 is connected to the conduction contact area 301 of the circuit board 3 by the connection portion 5.

In the present embodiment, the connection portion 5 may include the resilient structure, and the resilient structure may keep the first end of the probe 2 in resilient contact with the conduction contact area 301 of the circuit board 3 to ensure a good connection.

Furthermore, the resilient structure may include, but is not limited to, a spring plate 201 or a protrusion 202, etc., and any structure that can keep the first end of the probe 2 in resilient contact with the conduction contact area 301 of the circuit board 3 is acceptable.

In some embodiments, referring to FIG. 3, the first end of the probe 2 may be provided with a spring plate 201, and the probe 2 may be connected to the conduction contact area 301 of the circuit board 3 by the spring plate 201.

In the present embodiment, the probe 2 may be connected to the conduction contact area 301 of the circuit board 3 by the spring plate 201 provided on the first end of the probe 2 to realize signal transmission. The spring plate 201 may has a certain degree of flexibility and elastic deformation ability, which may ensure good contact between the first end of the probe 2 and the conduction contact area 301 of the circuit board 3, enhance the reliability of the connection between the probe 2 and the circuit board 3, and reduce the risk of connection failure.

Furthermore, the spring plate 201 may be a three-dimensional spring plate, which may be defined on the first end of the probe 2 by a cutting manner, and the cutting manner may include a laser cutting, a physical cutting, and so on. Specifically, three sides may be cut on the first end of the probe 2, be separated from the first end of the probe 2, and be bend into a certain shape, such as an arch, a wedge, etc., leaving one side still connected to the first end of the probe 2. The number of the spring plate 201 may be two or more.

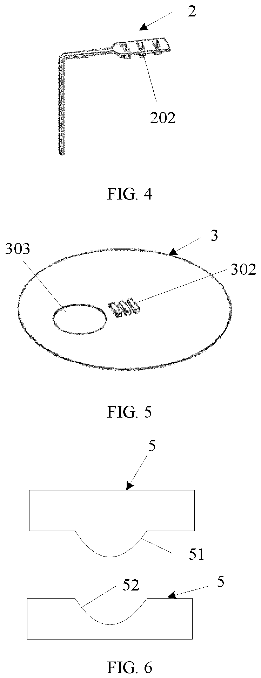

In some embodiments, referring to FIG. 4, the first end of the probe 2 may be provided with a protrusion 202, and the probe 2 may be connected to the conduction contact area 301 of the circuit board 3 by the protrusion 202

In the present embodiment, the probe 2 may be connected to the conduction contact area 301 of the circuit board 3 by the protrusion 202 provided on the first end of the probe 2 to realize signal transmission. The protrusion 202 may have a certain flexibility and elastic deformation ability, which may ensure good contact between the first end of the probe 2 and the conduction contact area 301 of the circuit board 3, enhance the reliability of the connection between the probe 2 and the circuit board 3, and reduce the risk of connection failure.

Furthermore, the protrusion 202 may be a three-dimensional protrusion, which may be defined on the first end of the probe 2 by embossing. Specifically, the three-dimensional protrusion may be embossed on the first end of the probe 2 by IME (In Mold electronics) technology. The number of the protrusion 202 may be two or more.

Furthermore, the first end of the probe 2 may be provided with both the spring plate 201 and the protrusion 202. Specifically, the first end of the probe 2 may be provided with two spring plates 201 and one protrusion 202 between the two spring plates 201, or the first end of the probe 2 may be provided with two protrusions 202 and one spring plate 201 between the two protrusions 202.

In some embodiments, the connection portion 5 may include a contact structure, and the first end of the probe 2 may be connected to the conduction contact area of the circuit board 3 by the contact structure.

In the present embodiment, the connection portion 5 may include the contact structure, and the contact structure may keep the first end of the probe 2 in good contact with the conduction contact area 301 of the circuit board 3.

Furthermore, the contact structure may include, but is not limited to, a convex contact 302 or the like, and any structure that can keep the first end of the probe 2 in good contact with the conduction contact area 301 of the circuit board 3 is acceptable.

In some embodiments, referring to FIG. 5, the conduction contact area 301 of the circuit board 3 may be provided with a convex contact 302, and the first end of the probe 2 may be connected to the conduction contact area 301 of the circuit board 3 by the convex contact 302.

In the present embodiment, the conduction contact area 301 of the circuit board 3 may be connected to the first end of the probe 2 by the convex contact 302 to realize signal transmission. The convex contact 302 may maintain a certain contact force with the first end of the probe 2 by an elastic deformation ability of material or structure of the convex contact 302, which may enhance the reliability of the connection between the probe 2 and the circuit board 3, and reduce the risk of connection failure.

Furthermore, the convex contact 302 may be realized by three-dimensional circuit technology, including LDS (Laser Direct Structuring) technology, IME (In Mold electronics) technology, and the like. The number of the convex contact 302 may be two or more.

Furthermore, the first end of the probe 2 may be provided with a spring plate 201, the conduction contact area 301 of the circuit board 3 may be provided with a convex contact 302, and positions of the spring plate 201 and the convex contact 302 may be aligned with each other or staggered with each other; or, the first end of the probe 2 may be provided with a protrusion 202, the conduction contact area 301 of the circuit board 3 is provided with a convex contact 302, and positions of the protrusion 202 and the convex contact 302 may be aligned with each other or staggered with each other. In this way, it may enhance the reliability of the connection between the probe 2 and the circuit board 3 and reduce the risk of connection failure.

In some embodiments, the connection portion 5 provided on the first end of the probe 2 may include a spring plate 201 and a protrusion 202, and the first end of the probe 2 is connected to the conduction contact area 301 of the circuit board 3 by the spring plate 201 and the protrusion 202, respectively. The connection portion 5 provided on the conduction contact area 301 of the circuit board 3 may include a convex contact 302, and the first end of the probe 2 is connected to the conduction contact area 301 of the circuit board 3 by the convex contact 302.

In the present embodiment, the probe 2 may be connected to the conduction contact area 301 of the circuit board 3 by the spring plate 201 and the protrusion 202 provided on the first end of the probe 2, and the conduction contact area 301 of the circuit board 3 may be connected to the first end of the probe 2 by the convex contact 302, to realize signal transmission. The spring plate 201 and the protrusion 202 may have a certain flexibility and elastic deformation ability, and the convex contact 302 may maintain a certain contact force with the first end of the probe 2 by the elastic deformation ability of material or structure of the convex contact 302, which may ensure a good contact between the first end of the probe 2 and the conduction contact area 301 of the circuit board 3, enhance the reliability of the connection between the probe 2 and the circuit board 3, and reduce the risk of connection failure.

Furthermore, the number of any of the spring plate 201, the protrusion 202, and the convex contact 302 may be one or more. Positions of the spring plate 201, the protrusion 202, and the convex contact 302 may be staggered with each other, and the positions of the spring plate 201 and the protrusion 202 may also be aligned with the position of the convex contact 302. It could be understood by one killed in the art that the number and the positions of the spring plate 201, the protrusion 202, and the convex contact 302 may be flexibly set according to actual requirements.

**In** some embodiments, referring to FIG. 6, the connection portion 5 provided on the first end of the probe 2 may include a first matching structure 51, and the connection portion 5 provided on the conduction contact area 301 of the circuit board 3 may include a second matching structure 52, and the first matching structure 51 may be matched and connected to the second matching structure.

**In** the present embodiment, the connection portion 5 may include the first matching structure 51 or the second matching structure 52, the first matching structure 51 may be provided on the first end of the probe 2, the second matching structure 52 may be provided on the conduction contact area 301 of the circuit board 3, and it may be realized that good contact between the first end of the probe 2 and the conduction contact area 301 of the circuit board 3 by cooperative connection of the first matching structure 51 and the second matching structure 52.

Furthermore, the first matching structure 51 may include a convex structure and the second matching structure 52 may include a recessed structure; or the first matching structure 51 may include a recessed structure and the second matching structure 52 may include a convex structure. It may be realized that tight contact between the first end of the probe 2 and the conduction contact area 301 of the circuit board 3 by the convex structure and the recessed structure. The number and specific structures of the convex structure and the recessed structure may not be limited, and it is sufficient that the convex structure and the recessed structure match with each other.

In some embodiments, referring to FIG. 5, the circuit board 3 may be provided with a first through hole 303, a second end of the probe 2 that is not connected to the conduction contact area 301 may be bent relative to the first end of the probe 2, and the second end of the probe 2 may pass through the first through hole 303 of the circuit board 3 when the first end of the probe 2 is connected to the conduction contact area 302 of the circuit board 3.

In the present embodiment, the first end of the probe 2 is configured to be connected to the conduction contact area 302 of the circuit board 3 for signal transmission, and the second end of the probe 2 may be bent relative to the first end of the probe 2 and pass through the first through hole 303 of the circuit board 3 to facilitate that the second end of the probe 2 can be implanted into the subcutaneous tissue of the object to be detected.

Furthermore, the second end of the probe 2 may pass through the first through hole 303 of the circuit board 3 at a central or eccentric position.

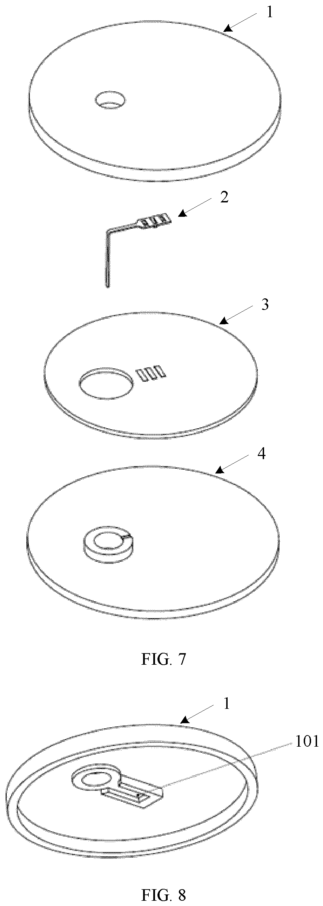

In some embodiments, referring to FIG. 7, the analyte sensor 100 may further include a first cover plate 1 and a second cover plate 4, the probe 2 and the circuit board 3 may be provided between the first cover plate 1 and the second cover plate 4, and the first cover plate 1 and the second cover plate 4 may be detachably connected with each other and configured to fix the probe 2 and the circuit board 3.

In the present embodiment, the first cover plate 1 and the second cover plate 4 may be detachably connected with each other, and when the first cover plate 1 and the second cover plate 4 are connected with each other, the probe 2 and the circuit board 3 may be fixed to ensure good contact between the probe 2 and the circuit board 3.

Furthermore, in other embodiments, the first cover plate 1 and the second cover plate 4 may be connected by screwing, hinging, clamping, and the like.

Furthermore, referring to FIG. 8, the first cover plate 1 may be provided with a clamping slot 101, which is configured to accommodate and fix the first end of the probe 2. When the first cover plate 1 and the second cover plate 2 are connected, the first end of the probe 2 accommodated in the clamping slot 101 may be connected to the conduction contact area 302 of the circuit board 3.

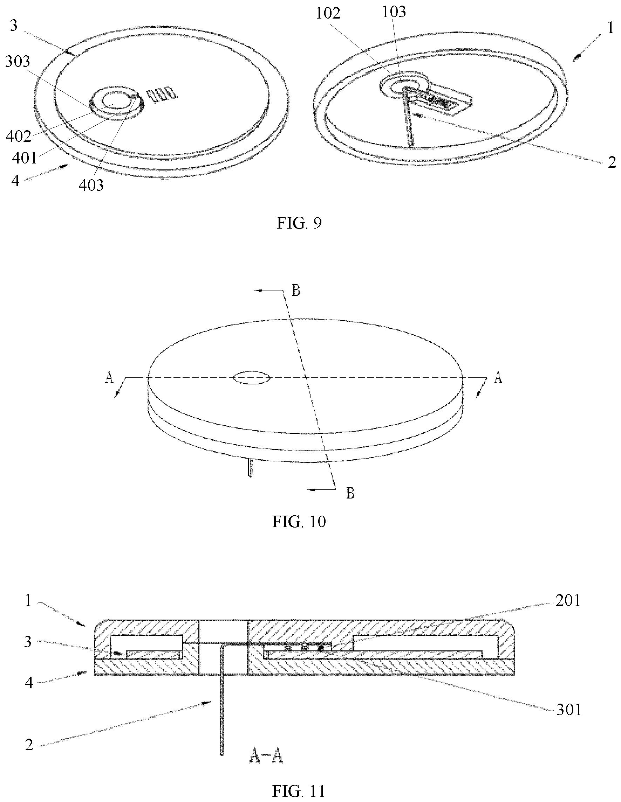

In some embodiments, referring to FIG. 9, the first cover 1 may be further provided with a first matching portion 102, the second cover 4 may be provided with a second matching portion 401, and the first matching portion 102 may be matched and connected to the second matching portion 401. The second matching portion 401 may pass through the first through hole 303, the first matching portion 102 may be provided with a second through hole 103, and the second matching portion 401 may be provided with a third through hole 402. When the first matching portion 102 is matched and connected to the second matching portion 401, the second through hole 103 and the third through hole 402 may be coaxially disposed, and the second end of the probe 2 may pass through the third through hole 402.

In the present embodiment, the first cover plate 1 may be matched and connected to the second cover plate 4 by the first matching portion 102 and the second matching portion 401, ensuring that the probe 2 is fixed and good contact between the probe 2 and the circuit board 3. The second matching portion 401 may pass through the first through hole 303 of the circuit board 3, so that the circuit board 3 may be fixed on the second cover plate 4. When the first matching portion 102 is matched and connected to the second matching portion 401, the second through hole 103 and the third through hole 402 may be coaxially disposed, and the second end of the probe 2 may pass through the third through hole 402, so that a user is able to directly observe the probe 2 from the second through hole 103 and the third through hole 402 when using the analyte sensor.

In some embodiments, referring to FIG. 9, the second matching portion 401 may further be provided with a groove 403, and a part of the probe 2 between the first end of the probe 2 and the second end of the probe 2 may be disposed in the groove 403.

In the present embodiment, since the probe 2 is provided with a certain thickness, the part of the probe 2 between the first end of the probe 2 and the second end of the probe 2 may be disposed in the groove 403 of the second matching portion 401, which facilitates fixing the probe 2 better.

Furthermore, a groove depth of the groove 403 may be equal to or slightly less than the thickness of the probe 2 along a groove depth direction.

In some embodiments, the analyte sensor 100 may be assembled in the following manner. Referring to FIG. 8, the circuit board 3 may be fixed to the second cover plate 4 by the first through hole 303 and the second matching portion 401, the first end of the probe 2 may be fixed in the clamping slot 101 of the first cover 1, then the first cover plate 1 and the second cover plate 4 may be fixed by snapping the first cover plate 1 and the second cover plate 4 to each other via the first matching portion 102 and the second matching portion 401, and the part of the probe 2 between the first end of the probe 2 and the second end of the probe 2 may be disposed in the groove 403.

Furthermore, referring to FIG. 10 to FIG. 12, where the first end of the probe 2 is provided with the spring plate 201, the spring plate 201 may come into contact with the conduction contact area 301 of the circuit board 3, and maintain a certain contact force with the conduction contact area 301 of the circuit board 3 by the flexibility and elastic deformation ability of the spring plate 201 itself.

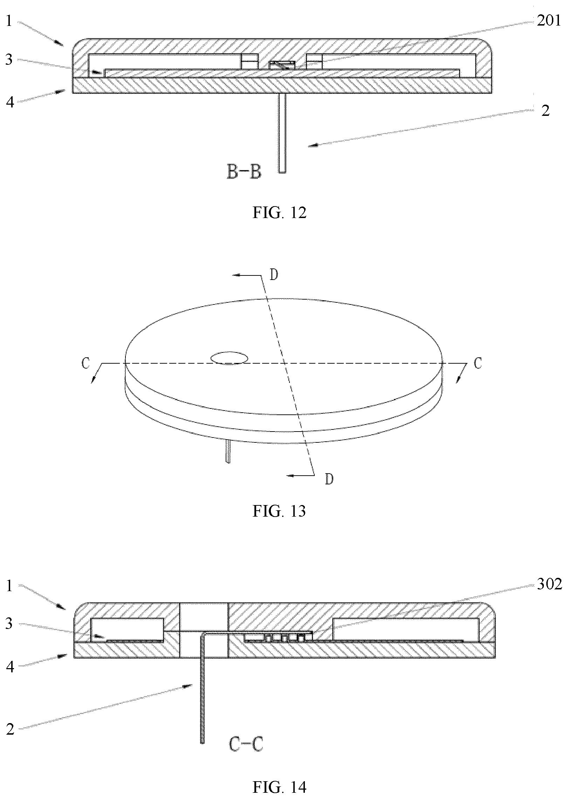

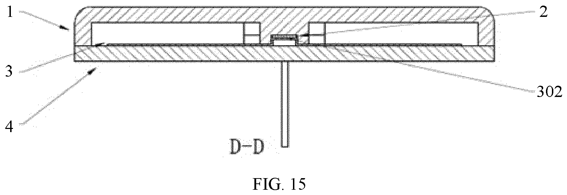

Referring to FIG. 13-FIG. 15, when the conductive contact area 301 of the circuit board 3 is provided with the convex contact 302, the convex contact 302 may come into contact with the first end of the probe 2 and maintain a certain contact force with the first end of the probe 2 by the flexibility and elastic deformation ability of the convex contact 302 itself.

The various technical features of the above-described embodiments may be combined in any combination, and for the sake of brevity of description, all possible combinations of the various technical features of the above-described embodiments have not been described, however, as long as there is no contradiction in the combinations of these technical features, these should all be considered to be within the scope of the present description as documented herein.

Those skilled in the art should realize that the above embodiments are only used to illustrate the present invention, and are not intended to be used as a limitation of the present invention, and as long as they are within the scope of the spirit of the substance of the present invention, appropriate alterations and variations of the above embodiments fall within the scope of the claimed protection of the present invention.

## Claims

1. An analyte sensor (100), **characterized by** comprising a probe (2) and a circuit board (3),
wherein a connecting portion (5) is provided on either or both of a first end of the probe (2) and a conductive contact area (301) of the circuit board (3), the first end of the probe (2) is connected to the conductive contact area (301) of the circuit board (3) by the connecting portion (5), and the connecting portion (5) and the first end of the probe (2) are integrally formed or the connecting portion (5) and the conductive contact area (301) of the circuit board (3) are integrally formed.

2. The analyte sensor (100) of claim 1, wherein the connecting portion (5) comprises a resilient structure and the first end of the probe (2) is connected to the conductive contact area (301) of the circuit board (3) by the resilient structure.

3. The analyte sensor (100) of claim 2, wherein the resilient structure comprises a spring plate (201), and the probe (2) is connected to the conductive contact area (301) of the circuit board (3) by the spring plate (201).

4. The analyte sensor (100) of claim 2 or claim 3, wherein the resilient structure comprises a protrusion (202), and the probe (2) is connected to the conductive contact area (301) of the circuit board (3) by the protrusion (202).

5. The analyte sensor (100) of any one of claims 1 to 4, wherein the connecting portion (5) comprises a contact structure, and the first end of the probe (2) is connected to the conductive contact area (301) of the circuit board (3) by the contact structure.

6. The analyte sensor (100) of claim 5, wherein the contact structure comprises a convex contact (302), and the first end of the probe (2) is connected to the conductive contact area (301) of the circuit board (3) by the convex contact (302).

7. The analyte sensor (100) of any one of claims 1 to 6, wherein the connecting portion (5) provided on the first end of the probe (2) comprises a first matching structure (51), the connecting portion (5) provided on the conductive contact area (301) of the circuit board (3) comprises a second matching structure (52), and the first matching structure (51) is matched and connected to the second matching structure (52).

8. The analyte sensor (100) of claim 7, wherein the first matching structure (51) comprises a convex structure and the second matching structure (52) comprises a recessed structure; or, the first matching structure (51) comprises a recessed structure and the second matching structure (52) comprises a convex structure.

9. The analyte sensor (100) of any one of claims 1 to 8, wherein the connecting portion (5) provided on the first end of the probe (2) comprises a spring plate (201) and a protrusion (202), and the first end of the probe (2) is connected to the conductive contact area (301) of the circuit board (3) by the spring plate (201) and the protrusion (202), respectively; and
the connecting portion (5) provided on the conductive contact area (301) of the circuit board (3) comprises a convex contact (302), and the first end of the probe (2) is connected to the conductive contact area (301) of the circuit board (3) by the convex contact (302).

10. The analyte sensor (100) of any one of claims 1 to 8, wherein the circuit board (3) is provided with a first through hole (303), a second end of the probe (2) which is not connected to the conductive contact area (301) of the circuit board (3) is bent relative to the first end of the probe (2), and the second end of the probe (2) passes through the first through hole (303) of the circuit board (3) when the first end of the probe (2) is connected to the conductive contact area (301) of the circuit board (3).

11. The analyte sensor (100) of any one of claims 1 to 10, further comprising a first cover plate (1) and a second cover plate (4), wherein
the probe (2) and the circuit board (3) are provided between the first cover plate (1) and the second cover plate (4), and the first cover plate (1) and the second cover plate (4) are detachably connected with each other and configured to fix the probe (2) and the circuit board (3).

12. The analyte sensor (100) of claim 11, wherein the first cover plate (1) is provided with a clamping slot (101), which is configured to accommodate and fix the first end of the probe (2).

13. The analyte sensor (100) of claim 11 or claim 12, wherein the first cover plate (1) is further provided with a first matching portion (102), the second cover plate (4) is provided with a second matching portion (401), and the first matching portion (102) is matched and connected to the second matching portion (401); and
the second matching portion (401) passes through the first through hole (303), the first matching portion (102) is provided with a second through hole (103), and the second matching portion (401) is provided with a third through hole (402); and when the first matching portion (102) is matched and connected to the second matching portion (401), the second through hole (103) and the third through hole (402) are coaxially disposed, and the second end of the probe (2) passes through the third through hole (402).

14. The analyte sensor (100) of claim 13, wherein the second matching portion (401) is further provided with a groove (403), and a part of the probe (2) between the first end of the probe (2) and the second end of the probe (2) is disposed in the groove (403).
